# EUROPEAN PATENT APPLICATION

(11) **EP 2 845 846 A1**
(43) Date of publication of application: **11.03.2015**
(21) Application number: 13866512.0
(22) Date of filing: 06.12.2013
(51) Int. Cl.: C07C 273/14, C07C 275/00

(54) **METHOD FOR PRODUCING AQUEOUS UREA AND METHOD FOR REMOVING AND RECOVERING TRIURET FROM AQUEOUS UREA**

(30) Priority: 29.06.2013 JP 2013137648
(71) Applicant: OPTY CO., LTD:, Mie-gun Mie 5108121 (JP)
(72) Inventor: INO Eiichi, Mie-gun Mie 510-8121 (JP)
(74) Representative: Cabinet Plasseraud
(86) International application number: PCT/JP2013/082873
(87) International publication number: WO 2014/207960

(57) **Abstract**

Provided is a method of efficiently removing triuret from urea water, a method of producing urea water of high purity and a method of collecting triuret from aqueous solution. Thus, a method of producing urea water comprises a step of preparing urea water by dissolving urea water in water, a step of making the urea water cloudy in a temperature range of -5°C or more and 17°C or less and a step of passing the urea water in a cloudy state through an ion exchange resin. Moreover, a method of removing triuret from urea water comprises a step of precipitating the triuret by cooling the urea water including urea and triuret as impurity in a temperature range of -5°C or more and 17°C or less and a step of passing the urea water in the temperature range through an ion exchange resin and capturing the precipitated triuret within the ion exchange resin.

## Description

### TECHNICAL FIELD

The present invention relates to a method for preparing urea water, a method for removing triuret from urea water and a method for collecting triuret from aqueous solution.

### BACKGROUND ART

Urea water has been used as technique for purifying NOx (nitrogen oxide) and exhaust gas purifying system etc. for a large passenger diesel car. The exhaust gas purifying system utilizes property that ammonium (NH₃) and nitrogen oxide are chemically reacted to be reduced into nitrogen (N₂) and water (H₂O). However, it is difficult to load ammonium on a car due to safety problem. Thus, urea water has been generally used.

Techniques for urea water are disclosed, for example, in patent document 1 and patent document 2 described below. In patent document 1, a method for producing urea water of high purity is disclosed, wherein urea water stock solution is treated in an acidic cation exchange resin and a basic anion exchange resin. In patent document 2, urea water having guanidine of 100 ppm or less, biuret of 2000 ppm or less, and hydrocarbon oil of 50 ppm or less and a denitration device are disclosed.

### Prior Art References

### Patent References

Patent reference 1: Japanese Patent Application Laid-Open Publication No. 2012-219040
Patent reference 2: Japanese Patent Application Laid-Open Publication No. 2007-145796

### DESCRIPTION OF THE INVENTION

### Problems to be Solved by the Invention

However, even though techniques disclosed in patent reference 1 and patent reference 2 are used, it was found that phenomenon such as cloudiness of urea water at low temperature occurs.

The inventor diligently studied on the cloudiness and found that cause of the cloudiness is not urea water itself but precipitation of triuret that is trimer of urea. The inventor studied more diligently on removing triuret and found that triuret is not removed by an ion exchange resin in a condition of urea water that is not cloudy and even if urea water passes through an ion exchange resin, urea water becomes cloudy again at low temperature. That is to say, it was found that it is difficult to obtain urea water of high purity by simply passing urea water through an ion exchange resin.

Thus, in order to solve the above-mentioned problem, it is an object of the present invention to provide a method of more efficiently removing triuret from urea water, a method of preparing urea water of high purity and a method of collecting triuret from aqueous solution.

### Means for Solving the Problems

The inventors found that even though urea water has been dissolved to be transparent, phenomenon such as cloudiness of urea water occurs at low temperature. The inventors also found that cause of the cloudiness was not urea itself but precipitation of triuret that is trimer of urea. In the case where urea water is not cloudy, triuret cannot be removed via an ion exchange resin. It was found that even though urea water is passing through an ion exchange resin, the urea water becomes cloudy again. Moreover, it was also found that triuret can be captured within an ion exchange resin in a cloudy state. Thus, the present invention is completed.

That is to say, a method of preparing urea water according to a first embodiment of the present invention comprises a step of producing urea water by dissolving urea water in water, a step of making urea water cloudy in a temperature range of -5°C or more and 17°C or less, and a step of passing urea water through an anion exchange resin in a state of cloudiness.

Furthermore, a method of preparing urea water according to a second embodiment of the present invention comprises a step of mixing urea that includes triuret as impurity with water, a step of making urea water cloudy by precipitating triuret in a temperature range of the urea water being -5°C or more and 17°C or less, and a step of capturing the precipitated triuret in an ion exchange resin by passing the urea water through the ion exchange resin in the temperature range.

Furthermore, a method of removing triuret from urea water according to a third embodiment of the present invention comprises a step of precipitating triuret by cooling urea water that includes urea and triuret as impurity in a temperature range of -5°C or more and 17°C or less and a step of capturing the precipitated triuret in an ion exchange resin by passing the urea water through the ion exchange resin in the temperature range.

Furthermore, a method of collecting triuret from aqueous solution according to a fourth embodiment of the present invention comprises a step of precipitating triuret by cooling aqueous solution that includes triuret in a temperature range of -5°C or more and 17°C or less and a step of capturing the precipitated triuret within an ion exchange resin by passing the aqueous solution through the ion exchange resin in the temperature range.

### Effects of the Invention

Hereinabove, it is provided that a more efficient method of removing triuret from urea water, a method of preparing urea water of high purity and a method of collecting triuret from aqueous solution.

### BRIEF DESCRIPTION OF THE DRAWINGS

Fig. 1 displays a flowchart for a method of preparing urea water according to one embodiment.
Fig. 2 displays a flowchart for a method of collecting precipitate in urea water according to another embodiment.
Fig. 3 displays infrared light absorption spectra for triuret, cyanuric acid, biuret and urea.
Fig. 4 displays X-ray diffraction results for triuret, cyanuric acid, biuret and urea.

### BEST MODE FOR CARRYING OUT THE PRESENT INVENTION

Hereinafter, embodiments of the present invention are described in detail. However, the present invention can be carried out within different embodiments and is not limited to embodiments and examples described below.

### (a method of preparing urea water and a method of removing triuret)

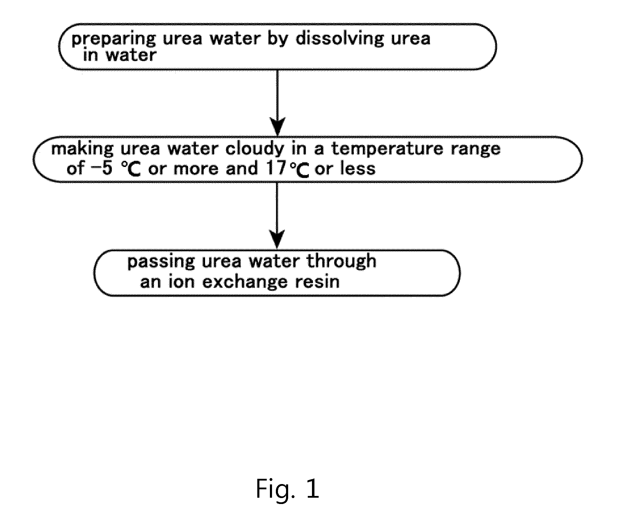

Fig. 1 shows a flowchart for a method of producing urea water (hereinafter, referred to as "the present producing method") according to the present invention. As shown in fig. 1, the present producing method comprises a step of producing urea water by dissolving urea water in water, a step of making the urea water cloudy in a temperature range of -5°C or more and 17°C or less, and a step of passing the urea water in a state of cloudiness through an anion exchange resin. More specifically, the present producing method comprises (1) a step of preparing urea water by mixing urea that includes triuret as impurity with water, (2) a step of precipitating triuret in a temperature range of -5°C or more and 17°C or less, and (3) a step of capturing the precipitated triuret within an ion exchange resin by passing the urea water through the ion exchange resin in the temperature range.

In the present invention, urea water is prepared by dissolving urea water in water. More specifically, (1) urea water is prepared by mixing urea that includes triuret as impurity with water. Herein, concentration of urea is not limited as far as function of urea water, for example reduction of NOx, is performed. Concentrations thereof include preferably 30 wt. % or more and 50 wt. % or less, more preferably 40 wt. % or less, and even more preferably 31 wt. % or more and 34 wt. % or less.

In the present step, triuret is included in urea as impurity. Herein, triuret means trimer of urea and a compound expressed by the following equation. Triuret is impurity inevitably included in the process of urea production. It is preferable that impurity is not included. However, in the present embodiment, when triuret of 0.045 wt. % or more is included, remarkable effect can be obtained for the present producing method. It is possible to obtain urea water that is not cloudy at low temperature by decreasing concentration of triuret in 0.005 wt. % or less.

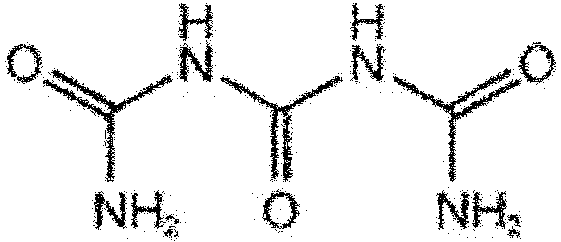

Furthermore, in the present producing method, urea water becomes cloudy by temperature thereof being -5°C or more and 17°C or less. More specifically, triuret is precipitated by temperature of the urea water being -5°C or more and 17°C or less. Triuret becomes substance that causes cloudiness at low temperature, and more specifically in 17°C or less. Function of urea water itself is not remarkably affected by the cloudiness. However, precipitation occurs in urea water due to cloudiness at low temperature. Thus, there are fears that clogging of a container or a pipe occurs by the precipitation remaining in the container and the pipe that receives urea water. Furthermore, there are fears that a nozzle that sprays urea or a filter for urea SCR system is clogged. Thus, in the present producing invention, methods for removing and collecting the precipitated triuret are provided by triuret being precipitated at low temperature. Herein, cloudiness is obtained by making a temperature range of 17°C or less, and cloudiness is remarkably obtained by making a temperature range of 12°C or less. Thus, it is a preferable temperature range of -5°C or more and 12°C or less.

Furthermore, the present producing method simultaneously performs a step of preparing urea water by dissolving urea water in water and a step of making cloudiness in a temperature range of -5°C or more and 17°C or less. More specifically, the present producing method simultaneously performs (1) a step of preparing urea water by mixing urea that includes triuret as impurity with water, (2) a step of precipitating triuret by putting urea water in a temperature range of -5°C or more and 17°C or less. In this case, the temperature range is maintained via heating reaction of water and urea water by a temperature range of water to be mixed being 10°C to 25°C higher than the desired temperature range.

Furthermore, in the present producing method, precipitate, i.e., triuret is captured within an ion exchange resin by passing cloudy urea water through an anion exchange resin, more specifically by passing urea water through an ion exchange resin in the temperature region. In the present producing method, it can be confirmed that even though triuret having no cloudiness cannot be removed by passing urea water through an ion exchange resin. In the present step, triuret can be captured through the ion exchange resin by intentionally making urea water cloudy. It is not clear that triuret becomes cause of cloudiness at low temperature. However, it is considered that triuret becomes bigger to such an extent that triuret agglomerates to make light scattering. Principle for removing triuret through an ion exchange resin in a cloudy state can be supposed. Capturing triuret by means of anion resin is considered to be effect of agglomeration of triuret due to alkali. Capturing triuret by means of cation resin is considered to be agglomeration of triuret in urea aqueous solution at low temperature and physical supplementation of triuret.

Furthermore, as mentioned above, an cation exchange resin can efficiently exchange cation that is slightly included in urea that contains ammonium, sulfuric acid calcium used as anti-cracking agent or sulfuric acid sodium. An anion exchange resin can efficiently agglomerate triuret as catalyst of OH group. Thus, triuret can be filtered.

Herein, an ion exchange resin may be any one of a cation exchange resin and an ion exchange resin, and may be combination thereof. When one of the cation exchange resin and the ion exchange resin is used, it is preferable that the amount of captured triuret by the anion exchange resin is higher than that of the captured triuret by the cation by three to four times. Herein, "cation exchange resin" is resin being capable of making ion exchange with cation that exists in solution such as water and is not limited thereto. Furthermore, conventional product can be used and, for example, AMBEREX100, AMBERITE IR120B, AMBERJET 1020, and the like made by Organo Co., Ltd. Herein, "anion exchange resin" is resin being capable of making ion exchange with anion that exists in solution such as water and is not limited thereto. Furthermore, conventional product can be used and, for example, AMBERLITE IRA410, AMBERLITE IRA402BL, AMBERJET 4010, AMBERJET 4002, and the like made by Organo Co., Ltd.

Hereinabove, it is realized that urea water of high purity can be produced without being cloudy even at low temperature by sufficiently removing triuret.

### (a method of collecting triuret)

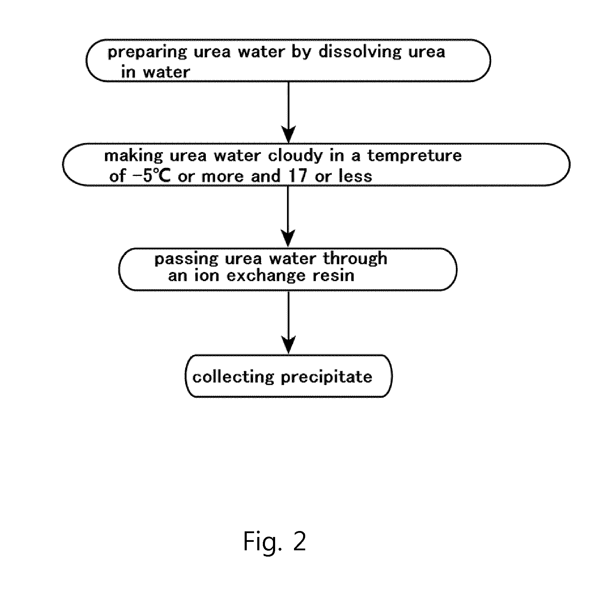

As mentioned above, in the present embodiment, triuret is sufficiently removed to provide a method of producing urea water of high purity without being cloudy at low temperature. Moreover, in the present embodiment, precipitate can be collected through an ion exchange resin. More specifically, a method of collecting triuret can be realized by including a step of cleaning ion exchange resin by using urea water to collect triuret. In fig. 2, this flow is shown.

In the present step, triuret that is easily captured from an ion exchange resin can be collected by cleaning the ion exchange resin. A method of cleaning the ion exchange resin is not specially limited. Cleaning is preferably performed by liquid and more preferably performed by water. Cleaning with water that is added by alcohol is preferable in view of efficiency for cleaning and collection.

The ion exchange resin after triuret is collected and cleaned can be repeatedly used in a step of removing triuret again because triuret is removed.

Hereinabove, according to the present embodiment, a method of collecting triuret from urea water can be provided.

### Examples

Hereinafter, a method of producing urea water according to the present embodiment, a method of removing triuret and a method of collecting triuret were performed. Effects thereof were confirmed. Details are descried.

### (confirming triuret)

At first, urea of 365kg is added into a plastic bowel (hereinafter, referred to as "stirring tank") having length of 1000mm, width of 900mm and depth of 1150 mm. The stirring tank is equipped with a stirring pump therein.

Then, an ion exchange resin where anion resin of 66% reproduced to OH type via NaOH and cation resin of 34% reproduced to H type via HCl (both anion resin and cation resin are produced by Dow Chemical) were added into a column having height of 900nm and diameter of 200mm(hereinafter, referred to as only "column"). Tap water of 758 litters was passing therethrough in a velocity of SV=20 and then stirred during 90 minutes to produce urea water with a concentration of 32.5 wt.% of 1123kg(i.e., 1030 litters).

When temperature of urea water was 12°C or less, it was confirmed that urea water became cloudy. When the cloudy urea water was standing, white precipitate was confirmed. Thus, the precipitate was cleaned with pure water to remove urea, and filtering was conducted. And then, natural drying process was conducted at room temperature.

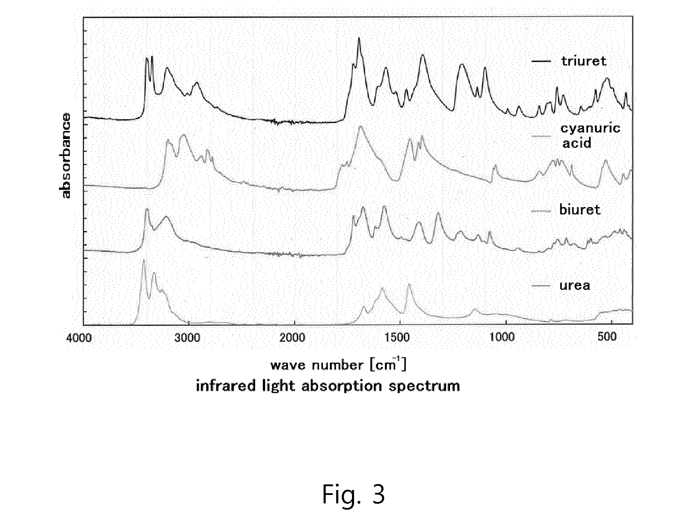

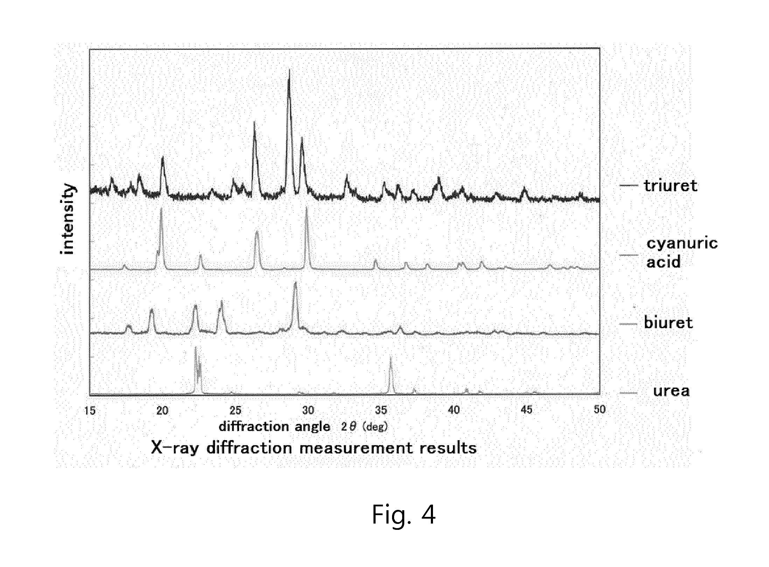

Infrared absorption spectrum and X-ray diffraction measurement were performed with respect to the white precipitate. It was confirmed that the white precipitate was triuret. Moreover, in fig. 3, infrared absorption spectra for triuret, cyanuric acid, biuret and urea are shown. In fig. 4, results of X-ray diffraction thereof are shown.

### (a method of removing triuret and a method of producing urea water)

Urea of 365kg was added into the stirring tank. The same ion exchange resin as mentioned-above configuration was poured into the column. Tap water of 758 litters was passing through in a velocity of SV=20. Stirring process during 90 minutes was performed and urea water in a concentration of 32.5 wt. % of 1123kg (1030 litters) was produced.

Next, temperature of urea water was adjusted to 10°C to make the urea water cloudy. Turbidity in a state of cloudiness was 30 NTU and was measured by digital turbidity (digital turbidity meters 500G made by KYORITSU CHEMICAL CHECK Lab., Corp.)

While cloudy urea water was passing through water in a velocity of SV=20, the cloudy urea water was transferred to sub-tank with the same size such as the stirring tank with respect to (1)a filter made by polypropylene having filtering precision of 25µ, diameter of 60mm and height of 250mm, (2)a filter poured by cation resin of 20 litters reproduced to H type via HCl (3)a filter poured by anion resin of 20 litters reproduced to OH type via NaOH, and (4) a filter made by polypropylene having filtering precision of 1µ, diameter of 60mm and height of 250mm.

The filters used in this method were separated from the stirring tank. And then the filters were stirred and cleaned with pure water of 40 litters. Triuret was captured in the ion exchange resin. It is considered that complete ion exchange was not performed. Triuret could be cleaned by pure water. In the below table, cloudiness, i.e., amount of triuret collected by cleaning is shown. The collected triuret was cleaned by pure water and amount of the triuret was obtained by naturally drying process for several days at room temperature. In this example, a plurality of same manipulation was conducted by varying temperature (for example, 8 to 14°C) and results thereof are shown.

**(table 1)**

| | **sample 1** | **sample 2** | **sample 3** | **sample 4** |
|---|---|---|---|---|
| **water temperature in cloudiness** | 8°C | 10°C | 12°C | 14°C |
| **amount of urea water to be produced** | 1,030 ℓ | 1,030 ℓ | 1,030 ℓ | 1,030 ℓ |
| **amount of urea** | 365kg | 365kg | 365kg | 365kg |
| **sampling amount for 25µ filter** | 1.1 g | 12g | 0.7g | 0.9g |
| **sampling amount for cation resin** | 6.2g | 5.1g | 5.3g | 4.8g |
| **sampling amount for anion resin** | 18.6g | 19.5g | 18.9g | 18.7g |
| **sampling amount for 1µ filter** | 4.4g | 3.4g | 2.9g | 3.3g |
| **sampling amount for a total amount** | 30.3g | 29.2g | 27.8g | 27.7g |

Thus, it was confirmed that a large amount of triuret could be removed. Especially, in the present example, effect of anion resin is extremely large. It was confirmed that anion resin had an ability of capturing triuret more than cation resin by about three to four times.

Urea water according to the present method did not cloudy even in a temperature range of -10°C or more and 17 or less, when triuret was sufficiently removed. In this case, turbidity was confirmed to be 20 NTU. Turbidity was measured by digital turbidity (digital turbidity meters 500G made by KYORITSU CHEMICAL CHECK Lab., Corp.) and this value was confirmed to be the same value such as turbidity of urea water having no-cloudiness at room temperature.

Hereinabove, urea water of high purity could be obtained. It was also confirmed that triuret could be removed and collected.

### (comparative example)

In the comparative example, the same condition was applied except that, when urea water is produced, temperature of water that is added into a stirring tank was 25°C and temperature of urea water was maintained over 25 to 33°C. The result thereof are shown in table 2. In this case, urea water was not cloudy.

**(table 2)**

| | **sample 5** | **sample 6** | **sample 7** | **sample 8** |
|---|---|---|---|---|
| **water temperature** | 25°C | 27°C | 32°C | 33°C |
| **amount of urea water to be produced** | 1.030 ℓ | 1,030 ℓ | 1,030 ℓ | 1,030 ℓ |
| **amount of urea** | 365kg | 365kg | 365kg | 365kg |
| **sampling amount for 25µ filter** | **unmeasurable** | **unmeasurable** | **unmeasurable** | **unmeasurable** |
| **sampling amount tur cation resin** | 0.2g | 0.3g | 0.1 g | 0.5g |
| **sampling amount for anion resin** | 1.2g | 2.0g | 1.8g | 2.8g |
| **sampling amount for 1µ filter** | 0.1g | 0.1g | 0.2g | 0.2g |
| **sampling amount for a total amount** | 1.5g | 2.4g | 2.1g | 3.5g |

From this result, when temperature of urea water was about 25°C (at room temperature), it was confirmed that triuret was stably dissolved into urea water and filtering was almost not performed by an ion exchange resin.

### (reconfirmation of removing triuret)

In the comparative example, the same process such that triuret was sufficiently removed was conducted with respect to urea water in which triuret was almost not removed. Results thereof are shown in table described below.

**(table 3)**

| | **sample 5'** | **sample 6'** | **sample 7'** | **sample 8'** |
|---|---|---|---|---|
| **water temperature in cloudiness** | 9°C | 10°C | 7°C | 9°C |
| **amount of urea water** to **be produced** | 1,030 ℓ | 1,030 ℓ | 1,030 ℓ | 1,030 ℓ |
| **amount of urea** | 365kg | 365kg | 365kg | 365kg |
| **sampling amount for 25µ filter** | 0.7g | 0.4g | 0.7g | 1.2g |
| **sampling amount tor cation resin** | 3.6g | 2.7g | 4.7g | 3.9g |
| **sampling amount for anion resin** | 10.9g | 8.5g | 10.6g | 10.8g |
| **sampling amount for 1µ filter** | 2.0g | 2.9g | 3.8g | 2.8g |
| **sampling amount for a total amount** | 17.2g | 14.5g | 19.8g | 18.7g |

Thus, it was confirmed that 20 NTU of turbidity was obtained without cloudiness even in a low temperature range of 12°C or less. In the present example, it was confirmed that triuret was sufficiently removed by making urea water cloudy and passing through an ion exchange resin.

### (cleaning of an ion exchange resin)

Herein, it was confirmed that a method of cleaning for an ion exchange resin in which triuret is captured within the above-mentioned example. Two cases were compared. One case is cleaning with water and another case is cleaning with methanol as alcohol being added into pure water. Results including specific amount and period for cleaning are shown in table 4.

**(table 4)**

| a cation exchange resin | | | cleaning with pure water (tap water possible) | | | |
|---|---|---|---|---|---|---|
| number of cleaning | kinds of cleaning fluid | amount of resin | amount of cleaning water | time of stirring and cleaning | rinsing timeé | result |
| the first | pure water | 20 ℓ | 40 ℓ | 30 minutes | 5 minutes | cloudiness remains |
| the second | pure water | 20 ℓ | 40 ℓ | 30 minutes | 5 minutes | cloudiness slightly remains |
| the third | pure water | 20 ℓ | 40 ℓ | 30 minutes | 5 minutes | cloudiness disappears and reusable |

| a cation exchange resin | | | cleaning by adding methanol into pure water | | | |
|---|---|---|---|---|---|---|
| number of cleaning | kinds of cleaning fluid | amount of resin | amount of cleaning water | time of stirrin and cleaning | rinsing time | result |
| the first | pure water plus methanol 20 volume% | 20 ℓ | 40 ℓ | 30 minutes | 5 minutes | cloudiness disappears and reusable |

| | | | | | | |
|---|---|---|---|---|---|---|
| a stirring machine: Nikko Co., Ltd. one axis free mode mixer (standard type) a stirring machine: Nikko Co., Ltd. one axis free mode mixer (standard type) | | | | | | |

Thus, when cleaning was performed with only water, cloudiness disappeared by about three times cleaning to be reusable. It was possible to largely decrease cleaning number by adding alcohol into water.

Although mentioned above, when an cation exchange rein that is already reproduced to H type by HCl has ion exchange, it is necessary to be H type by HCl for reuse. However, triuret can be removed by cleaning with only water in the ion exchange resin to which triuret is attached. It is considered that triuret is not attached to the cation exchange resin via ion exchange.

Furthermore, when an anion exchange rein that is already reproduced to OH type by NaOH has ion exchange, it is necessary to be OH type by NaOH for reuse. However, triuret can be removed by cleaning with only water in the ion exchange resin to which triuret is attached. It is considered that triuret is not attached to the anion exchange resin via ion exchange.

A ratio of triuret attached to the cation exchange resin and triuret attached to the anion exchange resin is about 1:3 or 1:4. The anion exchange resin has higher amount of filtering than the cation exchange resin. It was confirmed that the same result is obtained in the cases of changing order for urea water passing through the cation exchange resin and the anion exchange resin.

Thus, triuret can be cleaned by water. Since the ion exchange resin is porous medium, it takes time for removal by triuret being incorporated into hole. It was confirmed that cleaning for triuret can be performed in a short time by mixing alcohol with water.

### INDUSTRIAL APPLICABILITY

The present invention is industrially applicable for a method of preparing urea water, a method of removing triuret and a method of collecting triuret.

## Claims

1. A method of producing urea water comprising:
a step of preparing urea water by dissolving urea water in water;
a step of making the urea water cloudy in a temperature range of -5°C or more and 17°C or less; and
a step of passing the urea water in a cloudy state through an ion exchange resin.

2. The method of producing urea water according to claim 1,
wherein the step of preparing urea water by dissolving urea water in water and the step of making the urea water cloudy in a temperature range of -5°C or more and 17°C or less are simultaneously performed.

3. A method of producing urea water comprising:
a step of preparing urea water by mixing urea including triuret as impurity with water;
a step of making the urea water cloudy in a temperature range of -5°C or more and 17°C or less by precipitating the triuret; and
a step of passing the urea water in a cloudy state in the temperature range through an ion exchange resin and capturing the precipitated triuret within the ion exchange resin.

4. The method of producing urea water according to claim 3,
wherein the step of preparing urea water by mixing urea including triuret as impurity with water and the step of making the urea water cloudy in a temperature range of -5°C or more and 17°C or less by precipitating the triuret are simultaneously performed.

5. A method of removing triuret from urea water comprising:
a step of making urea water cloudy by cooling urea water including urea and triuret as impurity in a temperature range of -5°C or more and 17°C or less to precipitate the triuret; and
a step of passing the urea water in a cloudy state in the temperature range through an ion exchange resin and capturing the precipitated triuret within the ion exchange resin.

6. The method of removing triuret from urea water according to claim 5 further comprising:
a step of removing the triuret from the ion exchange resin by cleaning the ion exchange resin with water, the urea water passing through the ion exchange resin.

7. A method of collecting triuret from urea water comprising:
a step of precipitating triuret by cooling urea water including the triuret in a temperature range of -5°C or more and 17°C or less; and
a step of capturing the precipitated triuret within an ion exchange resin by passing the urea water in the temperature range through the ion exchange resin.
